# EUROPEAN PATENT APPLICATION

(11) **EP 4 487 777 A1**
(43) Date of publication of application: **08.01.2025**
(21) Application number: 22965407.4
(22) Date of filing: 14.11.2022
(51) Int. Cl.: A61B 5/24

(54) **SIGNAL COLLECTION SYSTEM**

(71) Applicant: Shenzhen Shokz Co., Ltd., Shenzhen, Guangdong 518108 (CN)
(72) Inventor: ZHOU, Xin, Shenzhen, Guangdong 518108 (CN); LI, Meiqi, Shenzhen, Guangdong 518108 (CN); SU, Lei, Shenzhen, Guangdong 518108 (CN); ZHANG, Yuxiang, Shenzhen, Guangdong 518108 (CN); LIU, Jia, Shenzhen, Guangdong 518108 (CN); ZHU, Huijin, Shenzhen, Guangdong 518108 (CN); NING, Yixuan, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2022/131703
(87) International publication number: WO 2024/103214

(57) **Abstract**

Embodiments of the present disclosure provide a signal acquisition system including a wearable body; a plurality of electrodes fixed on the wearable body and in contact with the skin of a user, and a processing circuit. The plurality of electrodes include a first electrode set and a second electrode set, and the first electrode set is configured to acquire a physiological signal and the second electrode set is configured to acquire a detection signal. The processing circuit configured to eliminate, based on the detection signal, motion artifacts in the physiological signal.

## Description

### TECHNICAL FIELD

The present disclosure relates to the field of signal acquisition, and in particular, to signal acquisition systems.

### BACKGROUND

Signal acquisition systems, widely used in physiological detection, disease diagnosis, experimental research, and other fields, can acquire data related to a user's physical condition by acquiring physiological signals. For example, the signal acquisition systems can detect and utilize information from an electrocardiogram signal to reflect the working status of the human heart. However, when the signal acquisition systems acquire the physiological signals, some interfering signals are often generated by movements or shakings of the human body that occurs during the signal acquisition process, which result in that the acquired physiological signals include motion artifacts, and a poorer quality of the physiological signals. Thus, it is difficult to accurately reflect the user's physical condition. Therefore, it is desired to provide signal acquisition systems that reduce the interference of motion artifacts and improve the quality of the acquired physiological signals.

### SUMMARY

One of the embodiments of the present disclosure provides a signal acquisition system comprising: a wearable body, a plurality of electrodes, and a processing circuit. The plurality of electrodes may be fixed on the wearable body and in contact with the skin of a user. The plurality of electrodes may include a first electrode set and a second electrode set, the first electrode set may be configured to acquire a physiological signal and the second electrode set may be configured to acquire a detection signal. The processing circuit may be configured to eliminate, based on the detection signal, motion artifacts in the physiological signal.

In some embodiments, the first electrode set may include two first electrodes arranged at interval, and the second electrode set may include two second electrodes arranged close to the two first electrodes, respectively. The physiological signal may be a first physiological signal, the detection signal may be a second physiological signal, and the processing circuit may be configured to eliminate, based on the second physiological signal, the motion artifacts in the first physiological signal.

In some embodiments, conductivities of materials of the two second electrodes may be different.

In some embodiments, heights of protrusions of the two second electrodes relative to the skin of the user may be different along a direction perpendicular to the skin surface of the user.

In some embodiments, hardness of the materials of the two second electrodes may be different, or folding degrees of the materials of the two second electrodes may be different.

In some embodiments, areas of the two second electrodes may be different.

In some embodiments, the first electrode set may include two first electrodes arranged at interval, and the second electrode set may include a second electrode arranged close to one of the two first electrodes. The second electrode and the one of the two first electrodes close to the second electrode may be configured to acquire the detection signal.

In some embodiments, a conductivity of a material of each second electrode may be weaker than a conductivity of a material of a first electrode corresponding to the second electrode.

In some embodiments, along a direction perpendicular to the skin surface of the user, a height of a protrusion of each second electrode relative to the wearable body may be less than a height of a protrusion of a first electrode corresponding to the second electrode relative to the wearable.

In some embodiments, a hardness of a second material of each second electrode may be greater than a first material of a first electrode corresponding to the second electrode, or a folding degree of the second material of each second electrode may be greater than a folding degree of the first material of the first electrode corresponding to the second electrode.

In some embodiments, a contact area of each second electrode with the skin may be less than a contact area of a first electrode corresponding to the second electrode with the skin.

In some embodiments, the first electrode set may include two first electrodes arranged at interval, and the second electrode set may include two second electrodes arranged close to the two first electrodes, respectively. The detection signal may reflect a contact impedance between the two first electrodes and the skin of the user.

In some embodiments, the signal acquisition system may further include an excitation source electrically connected to the two second electrodes. The excitation source may be configured to provide an excitation signal.

In some embodiments, a frequency of the physiological signal may be within a range of 20 Hz-400 Hz, and a frequency of the excitation signal may be not less than 250 Hz.

In some embodiments, a difference between the frequency of the excitation signal and an integer multiple of 50 Hz may be not less than 1 Hz; or a difference between the frequency of the excitation signal and an integer multiple of 60 Hz may be not less than 1 Hz.

In some embodiments, he frequency of the excitation signal may be higher than a frequency range of the physiological signal.

In some embodiments, the detection signal and the physiological signal may be acquired separately at different time periods.

In some embodiments, a minimum distance between an edge of each second electrode and an edge of a first electrode corresponding to the second electrode may be less than 10 cm.

In some embodiments, each second electrodes may be inelastically connected to a first electrode corresponding to the second electrode, and a ratio of a difference to a movable distance of the first electrode corresponding to the second electrode on a surface parallel to the skin surface may be not greater than 50%, the difference being a difference between a movable distance of the second electrode and the movable distance of the first electrode corresponding to the second electrode on the surface parallel to the skin surface.

In some embodiments, the processing circuit may be configured to perform the following operations.

The physiological signal may be obtained by differentially processing signals acquired by the two first electrodes.

The detection signal may be obtained by differentially processing signals acquired by the two second electrodes.

Based on the detection signal, the motion artifacts in the physiological signal may be eliminated.

In some embodiments, the signal acquisition system may also include an inertial sensor. The inertial sensor may be arranged on a side of the first electrode set depart from the skin of the user, and the inertial sensor may be configured to measure motion artifacts of the first electrode set.

One of the embodiments of the present disclosure further provides a signal acquisition system comprising a wearable body, a plurality of electrodes, an excitation source, and a processing circuit. The plurality of electrodes may be fixed on the wearable body and in contact with the skin of a user, and the plurality of electrodes may include two electrodes arranged at interval for acquiring a physiological signal. The excitation source may be electrically connected to the two electrodes, and the excitation source may be configured to provide an excitation signal for generating a detection signal reflecting a contact impedance between the two electrodes and the skin of the user. The processing circuit may be configured to eliminate, based on the detection signal, motion artifacts in the physiological signal.

In some embodiments, a frequency of the physiological signal may be within a range of 20 Hz-400 Hz, and a frequency of the excitation signal may be not less than 250 Hz.

In some embodiments, a difference between a frequency of the excitation signal and an integer multiple of 50 Hz may be not less than 1 Hz; or a difference between the frequency of the excitation signal and an integer multiple of 60 Hz may be not less than 1 Hz.

In some embodiments, a frequency of the excitation signal may be higher than a frequency range of the physiological signal.

In some embodiments, the detection signal and the physiological signal may be acquired separately at different time periods.

### BRIEF DESCRIPTION OF THE DRAWINGS

FIG. 1 is a block diagram illustrating an exemplary signal acquisition system according to some embodiments of the present disclosure;
FIG. 2 is a schematic diagram illustrating an exemplary signal acquisition system according to some embodiments of the present disclosure;
FIG. 3 is a schematic diagram illustrating an exemplary signal acquisition system according to some embodiments of the present disclosure;
FIG. 4 is a schematic diagram illustrating a structure of an exemplary signal acquisition system according to some embodiments of the present disclosure;
FIG. 5 is a schematic diagram illustrating a structure of an exemplary signal acquisition system according to some other embodiments of the present disclosure;
FIG. 6 is a block diagram illustrating an exemplary signal acquisition system according to some embodiments of the present disclosure;
FIG. 7A is a schematic diagram illustrating a fluctuation relationship between a contact impedance and an electromyographic signal according to some embodiments of the present disclosure;
FIG. 7B is a schematic diagram illustrating a fluctuation relationship between a contact impedance and an electromyographic signal according to some embodiments of the present disclosure; and
FIG. 8 is a block diagram illustrating an exemplary signal acquisition system according to some embodiments of the present disclosure.

### DETAILED DESCRIPTION

To more clearly illustrate the technical solutions related to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings. Unless obviously obtained from the context or the context illustrates otherwise, the same numeral in the drawings refers to the same structure or operation.

It should be understood that "system," "device," "unit," and/or "module" as used herein is a manner used to distinguish different components, elements, parts, sections, or assemblies at different levels. However, if other words serve the same purpose, the words may be replaced by other expressions.

As shown in the present disclosure and claims, the words "one," "a," "a kind," and/or "the" are not especially singular but may include the plural unless the context expressly suggests otherwise. In general, the terms "comprise," "comprises," "comprising," "include," "includes," and/or "including", merely prompt to include operations and elements that have been clearly identified, and these operations and elements do not constitute an exclusive listing. The methods or devices may also include other operations or elements.

The use of the words "first," "second", or the like in the present disclosure and the claims does not indicate any order or importance but is merely used to distinguish different components. The use of "first", "second", and similar terms in the present disclosure and the claims does not imply any order, number, or importance, but is merely used to distinguish between different components. Similarly, the words "a," "one", or the like do not indicate a quantitative limitation, but rather indicate the presence of at least one component. quantitative limitation. Unless otherwise noted, "anterior," "posterior," "lower," "rear," "lower," and/or "upper", and similar terms are for illustrative purposes only and are not limited to a location or a spatial orientation. In general, the terms "comprise," "comprises," "comprising," "include," "includes," and/or "including", merely prompt to include operations and elements that have been clearly identified, and these operations and elements do not constitute an exclusive listing.

Embodiments of the present disclosure describe a signal acquisition system. In some embodiments, the signal acquisition system may include a wearable body and a plurality of electrodes. The plurality of electrodes are fixed on the wearable body and in contact with the skin of a user when the wearable body is worn on the user to acquire a physiological signal of the user through the plurality of electrodes. In some embodiments, the plurality of electrodes may include a first electrode set and a second electrode set. The first electrode set includes two first electrodes arranged at interval to acquire a first physiological signal. The second electrode set include two first electrodes arranged respectively close to the two first electrodes to acquire a second physiological signal. In some embodiments, by designing the two electrode sets described above (e.g., the first electrode set and the second electrode set), the two electrode sets may acquire the differentiated first physiological signal and the second physiological signal. For example, the first physiological signal may contain a real physiological signal and motion artifacts, the second physiological signal may contain motion artifacts and a small amount of real physiological signals, or the second physiological signal may contain only the motion artifacts. The motion artifacts refer to interfering signals generated by the user's movements (e.g., head and/or limb swaying, etc.) during the signal acquisition process. Since the second electrodes are arranged close to the first electrodes, the second electrodes have a motion consistency with the first electrodes, and thus the physiological signal acquired by the second electrode set and the first electrode set have a strong correlation. In some embodiments, it may be assumed that the motion artifacts in the first physiological signal are roughly the same as the motion artifacts in the second physiological signal, and that the small amount of real physiological signals in the second physiological signal may be ignored. In some embodiments, the signal acquisition system may further include a processing circuit. The processing circuit may eliminate the motion artifacts in the first physiological signal based on the second physiological signal, to obtain the real physiological signal in the first physiological signal. The signal acquisition system described in the embodiments of the present disclosure may eliminate as many motion artifacts as possible in the acquired physiological signals, so that the ultimately obtained physiological signals are less intrusive and of high quality.

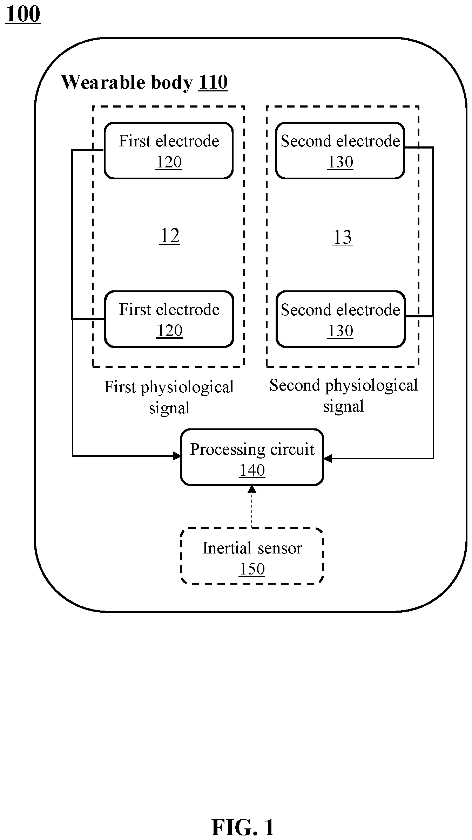

FIG. 1 is a block diagram illustrating an exemplary signal acquisition system according to some embodiments of the present disclosure. As shown in FIG. 1, a signal acquisition system 100 includes a wearable body 110, a first electrode set 12, a second electrode set 13, and a processing circuit 140. The first electrode set 12 may include two first electrodes 120, and the second electrode set 13 may include two second electrodes 130.

The wearable body 110 is worn on a user. In some embodiments, the wearable body 110 may be tops (e.g., a T-shirt, an undershirt, a tank top, a jacket, etc.) that is worn on the upper body of the user. In some embodiments, the wearable body 110 may be pants (e.g., pants, shorts, etc.) that are worn on the lower body of the user. In some embodiments, the wearable body 110 may also be a leg ring or a belt worn on the user's leg or waist, respectively. In some embodiments, the wearable body 110 may also include a smart bracelet, smart footwear, smart glasses, a smart helmet, a smart watch, a smart backpack, a smart accessory, or the like, or any combination thereof.

The electrodes (e.g., the first electrode 120 in the first electrode set 12 and the second electrode 130 in the second electrode set 13) refer to circuit elements for contacting other objects to input or derive a voltage (or a current). In some embodiments, the electrodes (e.g., the first electrode 120 in the first electrode set 12 and the second electrode 130 in the second electrode set 13) may be in contact with the skin for acquiring a physiological signal from the user. The physiological signal refers to a signal that may reflect a physical state of the user. In some embodiments, the physiological signal may include one or more signals such as a respiratory signal, an electrocardiogram (ECG) signal, an electromyographic (EMG) signal, a blood pressure signal, a blood oxygen signal, a temperature signal, or the like. The electrodes may be fixed on the wearable body 110 and remain in contact with the user's skin during the acquisition of the physiological signal. In some embodiments, the electrodes may be provided on the wearable body 110 relative to various parts of the human body, such as the calves, thighs, waist, back, chest, shoulders, neck, or the like.

For example, a plurality of electrodes for acquiring the ECG signal may be provided on the wearable body 110 (e.g., the waist, back, chest, hands, etc., of the human body) at different distances from the user's heart. For example, the two first electrodes 120 of the first electrode set 12 may be arranged at interval in the waist region of the human body, and the two second electrodes 130 in the second electrode set 13 are arranged close the two first electrodes 120, respectively. In some embodiments, to improve the similarity of motion artifacts in the ECG signal acquired by the two first electrodes (or the second electrodes), the wearable body 110 may symmetrically affix the two first electrodes (or the second electrodes) on two sides of the midsagittal plane of the human body. One of the two first electrodes 120 acquires a first potential and the other first electrode 120 acquires a second potential. There is a first potential difference between the first potential and the second potential (the first potential difference may be configured to generate a parameter reflecting a first ECG signal). One of the two second electrodes 130 acquires a third potential and the other second electrode 130 acquires a fourth potential. There is a second potential difference between the third potential and the fourth potential (the second potential difference may be configured to generate a parameter reflecting a second ECG signal).

As another example, a plurality of electrodes for acquiring the EMG signal may be provided at locations on the wearable body 110 that have large muscle groups (e.g., the back of the body, the waist, the legs, etc.). For example, the two first electrodes 120 of the first electrode set 12 may be arranged at interval on a piece of muscle, and the two second electrodes 130 in the second electrode set 13 are arranged close to the two first electrodes 120, respectively. In some embodiments, the two first electrodes 120 and the two second electrodes 130 may be arranged sequentially along a length direction of muscle fibers at the part. Different locations in the length direction of the muscle fibers have different potentials, and one of the two first electrodes 120 may acquire a first potential, and the other first electrode 120 may acquire a second potential. There is a first potential difference between the first potential and the second potential (the first potential difference may be configured to generate a parameter reflecting a first EMG signal). One of the two second electrodes 130 may acquire a third potential, and the other second electrode 130 may acquire a fourth potential. There is a second potential difference between the third potential and the fourth potential (the third potential difference may be configured to generate a parameter reflecting a second EMG signal).

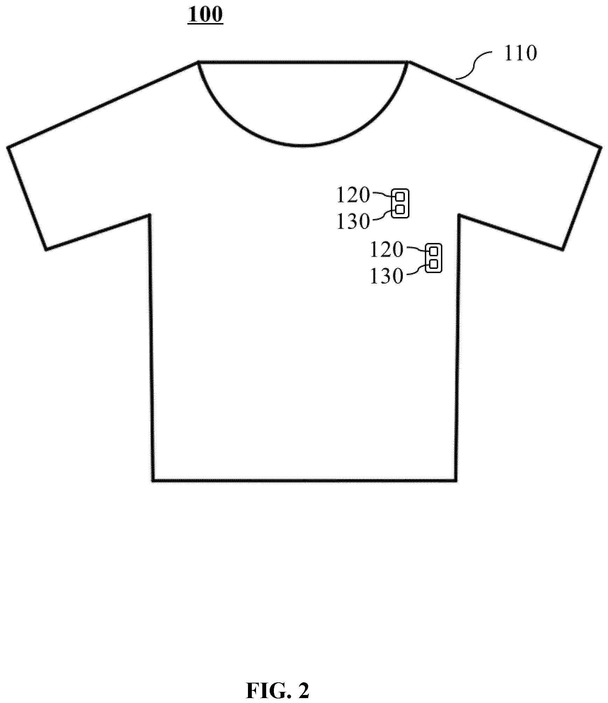

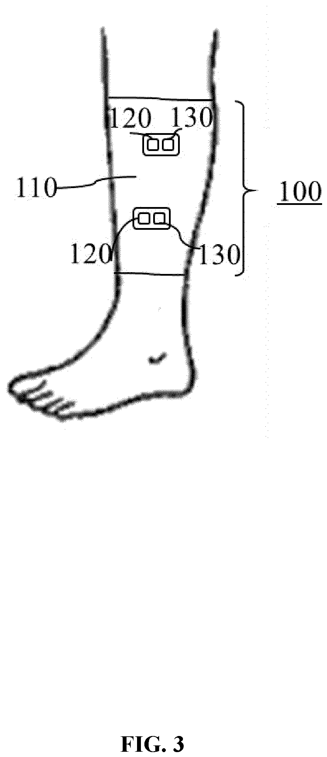

The following exemplary descriptions of the setting locations of the first electrodes 120 and the second electrodes 130 are described in conjunction with FIG. 2 and FIG. 3.

FIG. 2 is a schematic diagram illustrating an exemplary signal acquisition system according to some embodiments of the present disclosure. As shown in FIG. 2, the signal acquisition system 100 includes the wearable body 110, and the wearable body 110 is a top. The two first electrodes 120 are arranged at interval, and one of two second electrodes 130 is arranged close to one of the two first electrodes 120. As shown in FIG. 2, the two first electrodes 120 and the two second electrodes 130 may be arranged at a location of the upper garment corresponding to the chest of the human body.

FIG. 3 is a schematic diagram illustrating an exemplary signal acquisition system according to some embodiments of the present disclosure. As shown in FIG. 3, the wearable body 110 is a strap that is placed over the calf of the human body. In some embodiments, a first electrode 120 and a second electrode 130 arranged close to the first electrode 120 may be arranged on a side of the strap along a length direction of the muscle fibers. Another first electrode 120 and a second electrode 130 arranged close to the first electrode 120 may be arranged on another side of the strap along the length direction of the muscle fibers.

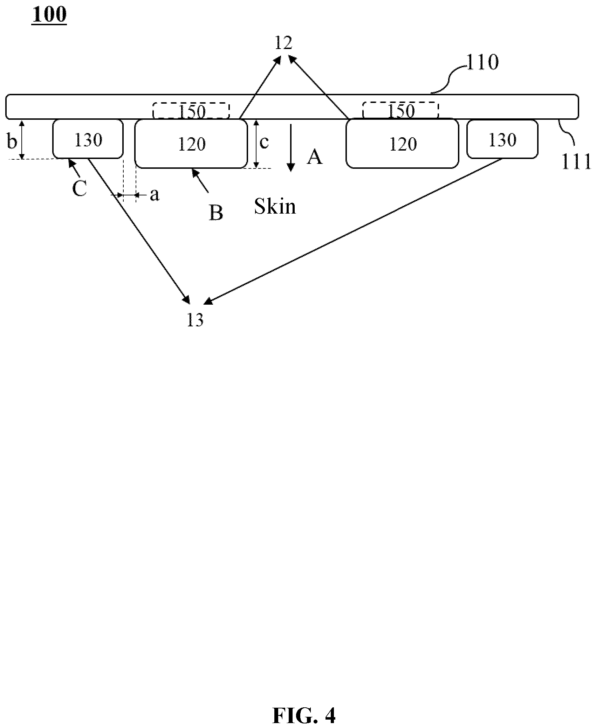

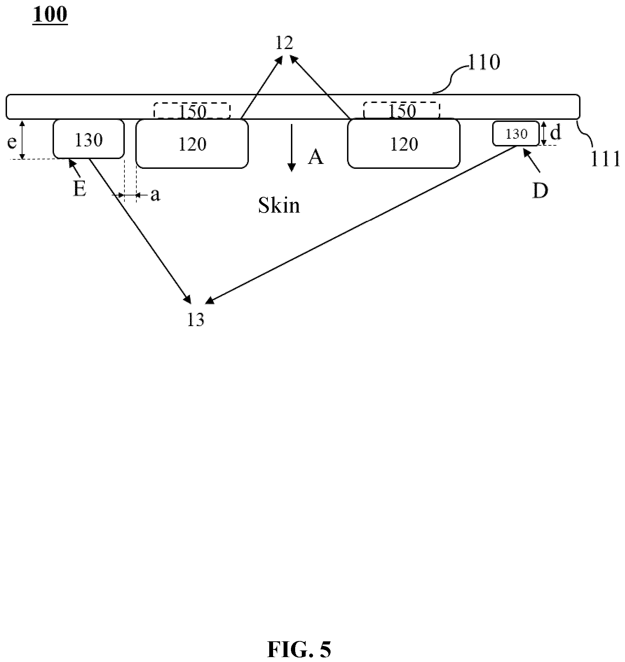

The processing circuit 140 may be configured to process signals. In some embodiments, the processing circuit 140 may be provided independently of the wearable body 110, and a plurality of electrodes may be communicatively connected to the processing circuit 140. In yet other embodiments, the processing circuit 140 may be fixed on the wearable body 110. In some embodiments, the first electrode set 12 (the two first electrodes 120) and the second electrode set 13 (the two second electrodes 130) may both be electrically connected to the processing circuit 140, and the processing circuit 140 receives the first potential and the second potential from the two first electrodes 120 and the third potential and the fourth potential from the two second electrodes 130. The processing circuit 140 may differentially process the first potential and the second potential to obtain a first potential difference for characterizing a physiological signal (e.g., a first physiological signal). The processing circuit 140 may differentially process the third potential and the fourth potential to obtain a second potential difference for characterizing the detection signal (e.g., a second physiological signal). In some embodiments, the processing circuit 140 may perform differential processing on the first physiological signals utilizing the second physiological signal, to remove motion artifacts from the first physiological signal. In some embodiments, the percentage of a real physiological signal and the motion artifacts in the first physiological signal may be different from the percentage of the real physiological signal and the motion artifacts in the second physiological signal by differentiating the design between the plurality of electrodes (e.g., the first electrode 120 and the second electrode 130, and/or the two second electrodes 130), to eliminate the motion artifacts in the first physiological signal based on the differentiated first physiological signal and the second physiological signal. More descriptions regarding differential design of the electrodes may be found in FIG. 4, FIG. 5, and related descriptions elsewhere in the present disclosure.

In some embodiments, the signal acquisition system 100 may also include an inertial sensor 150. The inertial sensor 150 is configured to measure the motion artifacts of the first electrode set 12. In some embodiments, the inertial sensor 150 may be provided on the first electrode set 12. For example, the inertial sensor 150 is provided at any one of the two first electrodes 120 (e.g., a side of the first electrode 120 that is away from the user's skin). At this time, motion states of the inertial sensor 150 and the first electrode 120 where the inertial sensor 150 is located may be considered to be consistent. Thus, a motion signal detected by the inertial sensor 150 may represent the motion state of the first electrode 120 where the inertial sensor 150 is located, and the detected motion signal may be configured to characterize the motion artifacts of the first electrode set 12. As another example, the inertial sensor 150 may be provided at each of the two first electrodes 120 (e.g., a side of each first electrode 120 that is away from the user's skin). At this time, each of the two inertial sensors 150 may detect two motion signals to indicate the motion state of the first electrode 120 where they are located. In some embodiments, the two motion signals detected by the two inertial sensors 150 may be processed (e.g., averaged, weighted averaged, etc.) to obtain the motion artifacts of the first electrode set 12.

In some embodiments, the inertial sensor 150 may be electrically connected to the processing circuit 140. In some embodiments, the signal acquisition system 100 may include a first electrode set 12, an inertial sensor 150 arranged on a side of the first electrode away from the user's skin, and a processing circuit 140. The first electrode set 12 includes two first electrodes 120 arranged at interval to collect the physiological signal (i.e., the first physiological signal). The inertial sensor 150 is configured to measure the motion signal of the first electrode set 12 as the motion artifacts of the first electrode set 12. The processing circuit 140 is configured to eliminate, based on the motion signal measured by the inertial sensor 150, the motion artifacts in the physiological signal acquired by the first electrode set 12. Since the principle of measuring the motion signal by the inertial sensor 150 is different from the principle of measuring the motion artifact contained in the physiological signal acquired by the first electrode set 12, the processing circuit 140 may eliminate the motion artifacts in the motion signal by preprocessing the motion signal and the physiological signal (e.g., normalizing the motion signal and the physiological signal) before performing differential processing. As another example, the motion artifacts in the motion signal are eliminated by preprocessing the motion signal and the physiological signal, performing a principal component analysis on both the motion signal and the physiological signal, removing principal components of the motion signal from the physiological signal, and then de-constructing. In some embodiments, the information acquisition system 100 may include a first electrode set 12, a second electrode set 13, an inertial sensor 150 arranged on the side of the first electrode away from the user's skin, and a processing circuit 140. The processing circuit 140 is configured to determine, based on the motion signal measured by the inertial sensor 150, a level of confidence of the second physiological signal configured to eliminate the motion artifacts in the first physiological signal. For example, a threshold may be preset in the processing circuit 140. If a difference between the motion signal measured by the inertial sensor 150 and the second physiological signal acquired by the second electrode set 13 is less than a preset threshold, the level of confidence of the second physiological signal is considered to be relatively high, the processing circuit 140 may perform differential processing on the first physiological signal based on the second physiological signal to eliminate the motion artifacts in the first physiological signal. If the difference between the motion signal and the second physiological signal is larger than the preset threshold, then the confidence level of the second physiological signal is considered to be relatively low, and the processing circuit 140 may send an instruction to re-acquire the second physiological signal to the second electrode set 13. As another example, the processing circuit 140 may perform differential processing (preprocessing followed by differential processing, as described above) on the first physiological signal based on the motion signal measured by the inertial sensor 150 to eliminate the motion artifacts in the first physiological signal.

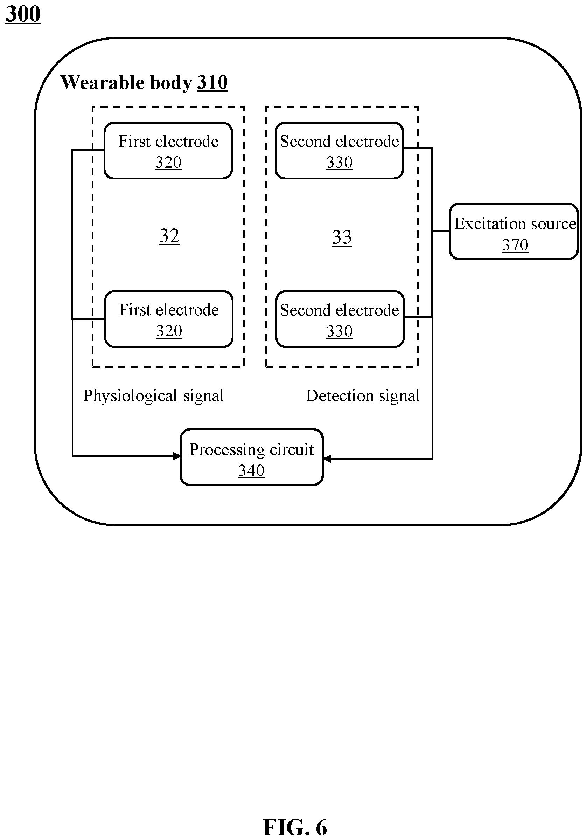

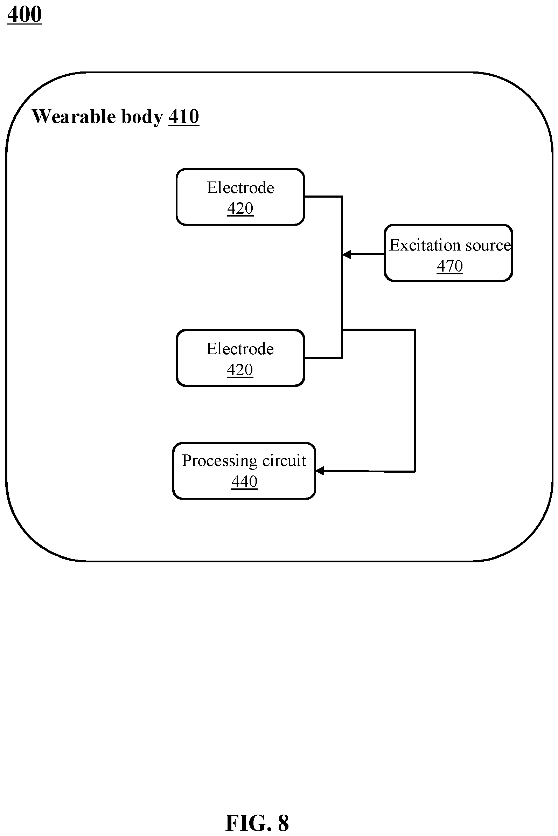

It should be noted that the signal acquisition system 100 may include a plurality of first electrode sets 12 and a plurality of second electrode sets 13. The plurality of first electrode sets 12 and the plurality of second electrode sets 13 are respectively fixed on the wearable body 110 corresponding to different parts of the human body to collect the physiological signal from different body parts of the user. It is also to be noted that the technical solution regarding the inertial sensor 150 may be applied to other embodiments of the present disclosure, for example, it may be applied to the signal acquisition system 100 shown in FIG. 4 and FIG. 5, the signal acquisition system 300 shown in FIG. 6, and the signal acquisition system 400 shown in FIG. 8.

In order to obtain accurate and high quality physiological signal, the differentiated first physiological signal and the second physiological signal may be obtained by differentiating designs between the plurality of electrodes (e.g., a differentiated design between the first electrode 120 and the second electrode 130 or a differentiated design between the two second electrodes 130). Exemplary illustrations of the differentiated design of the electrodes are shown below in conjunction with FIG. 4-FIG. 5. FIG. 4 is a schematic diagram illustrating a structure of an exemplary signal acquisition system 100 according to some embodiments of the present disclosure. FIG. 5 is a schematic diagram illustrating a structure of an exemplary signal acquisition system 100 according to some other embodiments of the present disclosure. As shown in FIG. 4 and FIG. 5, a plurality of electrodes may be fixed on a side of the wearable body 110 close to the user's skin and capable of contacting the skin. On the wearable body 110, the two first electrodes 120 may be provided at interval, and the two second electrodes 130 may be provided close to the first electrode 120, respectively. In some embodiments, the two first electrodes 120 and the two second electrodes 130 may be provided side-by-side, and the two second electrodes 130 may be located on opposite sides of the two first electrodes 120. In some embodiments, the two second electrodes 130 may be located between the two first electrodes 120. In some other embodiments, one of the second electrodes 130 may be located between the two first electrodes 120, and the other second electrode 130 may be located close to the first electrode 120 away from the other side of the first electrode 120. It is to be appreciated that the two first electrodes 120 and/or the two second electrodes 130 are not limited to being arranged side-by-side, as illustrated in FIG. 4 and FIG. 5, but may also be arranged in other manners. For example, the two first electrodes 120 may be arranged separately in any separated manner on surfaces parallel to the skin surface, and the second electrodes 130 provided close to each first electrode 120 may be provided at a distance at any location around the first electrodes 120, which is not limited in the present disclosure.

In some embodiments, a distance between the second electrode 130 and the first electrode 120 corresponding to the second electrode 130 refers to a minimum distance between an edge of the second electrode 130 and an edge of the first electrode 120 corresponding to the second electrode 130. As shown in FIG. 4 and FIG. 5, the distance between the second electrode 130 and the first electrode 120 corresponding to the second electrode 130 refers to a dimension a, which is a minimum distance between an edge of the second electrode 130 and an edge of its adjacent first electrode 120 close to the edge of the second electrode 130. To ensure that there is consistency in the movements of the second electrode set 13 and the first electrode set 12, in some embodiments, the distance may be less than 10 cm. For example, the distance may be less than 8 cm. As another example, the distance may be less than 6 cm. The "corresponding" described herein refers to a pair of first electrode 120 and second electrode 130 provided close to one another. That is, if the first electrode 120 and the second electrode 130 are provided close to each other, the first electrode 120 may be referred to as a first electrode corresponding to the second electrode 130, or the second electrode 130 may be referred to as a second electrode corresponding to the first electrode 120.

In some embodiments, to ensure that the movement of the second electrode set 13 is as consistent as possible with that of the first electrode set 12, the second electrode 130 may be in a physical connection with the corresponding first electrode 120. The physical connection as described in the present disclosure refers to a connection in a physical sense through a structure, material, or composite structural material. To avoid the influence of electrical signals between the first electrode 120 and the second electrode 130, the physical connection described herein is an insulating connection. In some embodiments, the connection between the second electrode 130 and the corresponding first electrode 120 may be realized by an insulating structure (e.g., a silicone layer). Any location of the second electrode 130 (e.g., a side surface, a lateral side, or localized region on a face, etc.) may be physically connected to any location of the first electrode 120 (e.g., a side face, a lateral side, or a localized region on a face, etc.) by the physical connection. In some embodiments, the second electrode 130 may be non-elastically connected (or rigidly connected) to the corresponding first electrode 120 to enhance the consistency of movement between the second electrode 130 and the corresponding first electrode 120. In some embodiments, a ratio of a difference to a movable distance of the first electrode corresponding to the second electrode on a surface parallel to the skin surface may be not greater than 50%. The difference is a difference between a movable distance of the second electrode 130 and the movable distance of the first electrode 120 corresponding to the second electrode 130 on a surface parallel to the skin surface. For example, the first electrode set 12 and the second electrode set 13 may move on the skin surface when the user is wearing the signal acquisition system 100, and when moving, the ratio of the difference to the movable distance of the first electrode corresponding to the second electrode on the surface parallel to the skin surface may be not greater than 45%. As another example, the ratio of the difference to the movable distance of the first electrode corresponding to the second electrode on the surface parallel to the skin surface may be not greater than 40%.

In order to reduce the interference of the motion artifacts and to improve the quality of the ultimately obtained physiological signal, the differentiated design of the first electrode 120 and the second electrode 130 may be configured to enhance a differentiation between the first physiological signal and the second physiological signal.

In some embodiments, the material of each second electrode 130 is less conductive than the material of the first electrode 120 corresponding to (e.g., physically connected) the second electrode 130. In this way, a percentage of the real physiological signal in the first physiological signals acquired by the first electrode set 12 is greater than a percentage of the real physiological signal in the second physiological signal acquired by the second electrode set 13. In some embodiments, when the conductivity of the material of the second electrode 130 is weaker than the conductivity of the material of the first electrode 120 corresponding to the second electrode 130 by a certain degree (e.g., the impedance of the material of the second electrode 130 differs from the impedance of the material of the corresponding first electrode 120 by a certain degree), the real physiological signal in the second physiological signals acquired by the second electrode set 13 may be so small as to be negligible. Thus, the second physiological signal may be considered to contain only motion artifacts. For example, at a size under an electrode protection, the impedance of the first electrode 120 is in a range of a few thousand ohms. When a difference between the impedance of the material of the first electrode 120 and the impedance of the material of the second electrode 130 reaches 1M ohm or more, motion artifact signals in the second physiological signal acquired by the second electrode set 13 becomes significantly more. When the difference between the impedance of the material of the first electrode 120 and the impedance of the material of the second electrode 130 reaches 100M ohm or more, the second physiological signals are acquired mainly with motion artifacts. At the same time, because the movements of the first electrode set 12 and the second electrode set 13 have consistency, the motion artifacts in the first physiological signal may be eliminated based on the second physiological signal to reduce the interference of the motion artifacts in the first physiological signal, to obtain a high-quality physiological signal. In some embodiments, the electrodes may be electrodes including a single material, such as metal fabric electrodes, conductive silicon electrodes, hydrogel electrodes, metal electrodes, or the like. For example, the material of each first electrode 120 may be a metal fabric, and the material of the second electrode 130 corresponding to the first electrode 120 may be electrically conductive silicon. The metal fabric electrodes having a lower electrical resistivity and greater electrical conductivity. In some embodiments, a difference in the materials of the electrodes affects not only the impedance of the electrodes, but also a contact impedance between the electrodes and the skin. For example, the material of each first electrode 120 may be a hydrogel, and the material of the second electrode 130 corresponding to the first electrode 120 may be a conductive silicon. The hydrogel is skin-friendly and stays moisturized as compared to the conductive silicon. Thus, the first electrode 120 has a small contact impedance and is more electrically conductive relative to the second electrode 130 corresponding to the first electrode 120. In some embodiments, the difference in the materials of the electrodes also affects a strength of a potential of the stratum corneum. For example, a silver chloride material has a smaller absolute value of the half-cell potential compared to a silver material with a smaller cuticle potential, and under the same other conditions, there is less of an effect of motion artifacts. In some embodiments, the differentiation of the first electrode 120 and the second electrode 130 may be achieved by different thicknesses of the same material. For example, as a thickness of an electrode of the metal fabric is within a certain range, the greater the thickness, the lower the impedance and the contact impedance with the skin, and the better the conductivity. In some embodiments, the material of each first electrode 120 and the material of the second electrode 130 corresponding to the first electrode 120 may both be the metallic fabric, but a thickness of the material of the first electrode 120 in a direction perpendicular to a skin direction (see the direction A shown in FIGs. 4 and 5) is greater than a thickness of the material of the second electrode 130 corresponding to the first electrode 120 in the direction A. As another example, since the greater a thickness of an electrode of the conductive silicon, the smaller its impedance and the better the conductivity, in some embodiments, the material of the first electrode 120 and the material of the second electrode 130 corresponding to the first electrode 120 may both be conductive silicon, but the thickness of the material of the first electrode 120 is greater than the thickness of the material of the second electrode 130 corresponding to the first electrode 120. In some embodiments, the material of the first electrode 120 and/or the material of the corresponding second electrode 130 may be a combination of different materials (e.g., laminated, combined, etc.). For example, the material of the first electrode 120 and the material of the second electrode 130 corresponding to the first electrode 120 are both composed of the metallic fabric and the conductive silicone, and the thickness of the metallic fabric in the material of the first electrode 120 is less than the thickness of the metallic fabric in the material of the second electrode 130 corresponding to the first electrode 120, and the thickness of the conductive silicon in the material of the first electrode 120 is greater than the thickness of the conductive silicon in the material of the second electrode 130 corresponding to the first electrode 120.

In some embodiments, the electrodes may be fixed to the wearable body 110 by means of gluing, snaps, Velcro, sewing, pressing, or the like, and the electrodes may have a protrusion relative to a direction of the wearable body 110 toward the skin surface. In some embodiments, in a direction along a direction perpendicular to the skin surface of the user, a height of each second electrode 130 relative to the wearable body 110 may be less than a height of the first electrode 120 corresponding to the second electrode 130 relative to the wearable body 110. The protrusion of the electrode in the present disclosure refers to a portion of the electrode that extends beyond a surface 111 of the wearable body 110 close to the skin. The height of the protrusion refers to a height of a portion of the electrode that extends beyond the surface 111 of the wearable body 110 close to the skin in a direction perpendicular to the skin surface of the user. As shown in FIG. 4, the height of each first electrode 120 relative to the protrusion of the wearable body 110 along the direction perpendicular to the skin surface (i.e., the direction A) is c. The height of the second electrode 130 corresponding to the first electrode 120 relative to the protrusion of the wearable body 110 along the direction perpendicular to the skin surface (i.e., the direction A) is b. To make the pressure between the second electrode 130 and the skin smaller than the pressure between the first electrode 120 corresponding to the second electrode 130 and the skin, so that the fitness of the second electrode 130 to the skin is worse relative to the first electrode 120, thus the proportion of the real physiological signal in the second physiological signal acquired by the second electrode set 13 is small. As shown in FIG. 4, the height b of the protrusion of each second electrode 130 relative to the wearable body 110 along the direction perpendicular to the skin surface (i.e., the direction A) may be smaller than the height c of the protrusion of the first electrode 120 corresponding to the second electrode 130 relative to the wearable body 110 along the direction perpendicular to the skin surface (i.e., the direction A). In this way, there is a differentiation between the first electrical signal acquired by the first electrode set 12 and the second electrical signal acquired by the second electrode set 13. In some embodiments, when a ratio of a difference to the height c of the protrusion of the first electrode 120 corresponding to the second electrode 130 is greater than a certain height threshold (e.g., 5%), , there is a significant difference between the first electrical signal acquired by the first electrode set 12 and the second electrical signal acquired by the second electrode set 13, and thus the second physiological signal may be considered to contain only motion artifacts. The difference is a difference between the height b of the protrusion of the second electrode 130 and the height c of the protrusion of the first electrode 120 corresponding to the second electrode 130. At the same time, there is a consistency in the motion of the first electrode set 12 and the second electrode set 13, the second physiological signal may be utilized to perform a differential processing on the first physiological signal to eliminate motion artifacts in the first physiological signal, thus reducing the interference of motion artifacts in the first physiological signal and obtaining a high-quality physiological signal. In some embodiments, in order to generate the differentiated first physiological signal and the second physiological signal, the height of the protrusion of each first electrode 120 may be within a range of 1 mm-10 cm, and the height of the protrusion of the second electrode 130 corresponding to the first electrode 120 may be within a range of 0 mm-5 mm.

In some embodiments, to produce the differentiated first physiological signal and the second physiological signal, the material of each second electrode 130 may be different than the material of the first electrode 120 corresponding to the second electrode 130. For example, a hardness of the material of each second electrode 130 may be greater than the material of the first electrode 120 corresponding to the second electrode 130 to allow the first electrode 120 to fit better relative to the skin of the user than the second electrode 130. Therefore, the percentage of the real physiological signal in the second physiological signal is less than that in the first physiological signal (i.e., the second physiological signal has a greater percentage of motion artifacts relative to the first physiological signal). In some embodiments, a folding degree of the material of each second electrode 130 may be greater than the material of the first electrode 120 corresponding to the second electrode 130 to allow for a better fit of the first electrode 120 relative to the skin of the user than the second electrode 130. Therefore, the percentage of the real physiological signal in the second physiological signal is less than that in the first physiological signal (i.e., the second physiological signal has a greater percentage of motion artifacts relative to the first physiological signal). It should be appreciated that the hardness or folding degree of the material is a comparison of measurements taken under the same measurement manner or the same measurement standard.

In some embodiments, the differentiated first physiological signal and the second physiological signal may be generated by differences in a contact impedance between the two electrode sets (the first electrode set 12 and the second electrode set 13) and the skin. In some embodiments, a differentiated contact impedance may be generated between the first electrode set 12 and the second electrode set 13 through a difference in the contact area of each first electrode 120 and the second electrode 130 corresponding to the first electrode 120 with the skin. For example, the first electrode 120 may have a first area with a skin contact surface (e.g., as shown on side B of FIG. 4), and the second electrode 130 corresponding to the first electrode 120 may have a second area with the skin contact surface (e.g., as shown on side C of FIG. 4). The second area of each second electrode 130 may be smaller than the first area of the first electrode 120 corresponding to the second electrode 130, to make the contact impedance between the first electrode 120 and the skin is less than the contact impedance between the second electrode 130 corresponding to the first electrode 120 and the skin. In this way, a percentage of the real physiological signal in the first physiological signal acquired by the first electrode set 12 is greater than a percentage of the real physiological signal in the second physiological signal acquired by the second electrode set 13. At the same time, a percentage of the motion artifacts in the second physiological signal is greater than a percentage of the motion artifacts in the first physiological signal. In some embodiments, to make the contact impedance between two electrode sets (the first electrode set 12 and the second electrode set 13) and the skin different, thereby generating the differentiated first physiological signal and the second physiological signal, the contact area of the first electrode 120 with the skin (e.g., the B surface shown in FIG. 4) may be within a range of 1 cm²-100 cm². The contact area of the second electrode 130 with the skin (e.g., the C surface shown in FIG. 4) may be within a range of 0.5 cm²-50 cm². In some embodiments, a shape of the B surface of the first electrode 120 or the C surface of the second electrode 130 may be a circle, triangular, hexagonal, and other regular or irregular shapes, and in practice, the shape of the B surface or C surface may depend on the shape of the site where the physiological signal is to be acquired. In some embodiments, a structure with a specific pattern or a pleated structure may be arranged on the C surface of each second electrode 130 to reduce the fit of the second electrodes 130 with the skin while reducing the contact area of the second electrode 130 with the skin, to achieve the purpose of differentiating from the first electrode 120.

It should be appreciated that the signal acquisition system 100 including four electrodes shown in FIGs. 1-3 is only exemplary and is not intended to limit the count of electrodes in the signal acquisition system 100. The count of electrodes in the signal acquisition system 100 may be any count capable of acquiring differentiated motion signals and physiological signals and is not limited herein. For example, the first electrode set 12 may include two first electrodes 120 and the second electrode set 13 may include only one second electrode 130 (i.e., the signal acquisition system 100 includes three electrodes). Two first electrodes 120 are configured to acquire the first physiological signal, and the second electrode 130 may be arranged close to one of the two first electrodes 120, and form an electrode pair with that first electrode 120 for acquiring the second physiological signal. It should be understood that when the second electrode set 13 may include only one second electrode 130, parameters of the second electrode 130 and the first electrode 120 corresponding to the second electrode 130 in the second electrode set, such as the material, the conductivity, the projection relative to the wearable body, the hardness, the folding degree, the contact area with the skin, etc., may be the same as parameters of each second electrode 130 and the first electrode 120 corresponding to the second electrode 130 in the second electrode set 13 when the second electrode set 13 may include two second electrodes 130, which will not be repeated herein. Furthermore, for example, on the basis of the above-described three electrodes, the signal acquisition system 100 may also include a reference electrode for acquiring a reference signal. As another example, in the signal acquisition system 100 including four electrodes as shown in FIGs. 1-3, two first electrodes 120 are configured to acquire the first physiological signals. Each second electrode 130 of the two second electrodes 130 and one of the two first electrodes 120 forms an electrode pair to acquire a second physiological signal and a third physiological signal respectively. The two second electrodes 130 may acquire a fourth physiological signal. In this way, the signal acquisition system 100 including four electrodes may acquire four sets of differentiated electrical signals. It should be appreciated that the first electrode set including two first electrodes 120 described herein is only exemplary and that the first electrode set may include more than two first electrodes 120. Correspondingly, in some embodiments, the second electrode set may include the same count of second electrodes 130 as the first electrodes 120. For example, the first electrode set includes three first electrodes 120 for acquiring the physiological signal; the second electrode set includes three second electrodes 130 for acquiring the detection signal. In some embodiments, the first electrode set may include more than two first electrodes 120, and the second electrode set may include at least one second electrode 130. For example, the first electrode set may include three first electrodes 120 for acquiring the physiological signal, and the second electrode set may include one second electrode 130 arranged close to one of the first electrodes 120 for acquiring the detection signal.

In some embodiments, the two second electrodes 130 in the second electrode set 13 may be the same to facilitate the production of a plurality of electrodes in the signal acquisition system 100. For example, each second electrode 130 may have the same conductivity, the same height of protrusion, the same hardness, the same folding degree, the same contact area with the skin, or the like, as shown in FIG. 4. In some embodiments, a differentiated design may be implemented on the two second electrodes 130 of the second electrode set 13 to reduce the interference of motion artifacts and to improve the quality of the physiological signal. When the two second electrodes 130 are different (e.g., different in one or any combination of the conductivity, the height of protrusion, the hardness, the folding degree, the contact area with the skin, etc.), a percentage of motion artifacts in the second physiological signal acquired by the second electrode set 13 is greater than a percentage of motion artifacts in the second physiological signal acquired by the same second electrodes 130. The differentiated first physiological signal and the second physiological signal may thus be obtained by the differentiated design of the two second electrodes 130 of the second electrode set 13 to utilize the second physiological signal to eliminate motion artifacts in the first physiological signal to reduce interference from the motion artifacts.

In some embodiments, the differentiation between the two second electrodes 130 may be designed similarly to the differentiation between the first electrode 120 and the second electrode 130 corresponding to the first electrode 120 described above. In some embodiments, the conductivity of the materials of the two second electrodes 130 may be different. For example, the two second electrodes 130 may be conductive silicon electrodes with different thicknesses. As another example, the materials of the two second electrodes 130 may be conductive silicon electrodes and fabric metal electrodes, respectively. In some embodiments, the heights of the projections of the two second electrodes 130 relative to the wearable body 110 along the direction A (see heights d and e shown in FIG. 5) may be different to allow for a different fit of the two second electrodes 130 relative to the skin of the user. In some embodiments, the hardness of the materials of the two second electrodes 130 may be different to allow for a different fit of the two second electrodes 130 relative to the skin of the user. In some embodiments, the folding degrees of the materials of the two second electrodes 130 may be different to allow for a different fit of the two second electrodes 130 relative to the skin of the user. In some embodiments, the contact areas of the two second electrodes 130 with the skin (see surface E and surface D shown in FIG. 5) may be different to allow for different contact impedances between the two second electrodes 130 and the skin.

In some embodiments, the signal acquisition system 100 may also include an inertial sensor 150, as shown in FIG. 4 and FIG. 5. The inertial sensor 150 is provided on a side of the first electrode 120 away from the skin of the user, so that the first electrode 120 and the inertial sensor 150 have a consistent motion state. It should be understood that FIG. 4 and FIG. 5 are only two embodiments of the signal acquisition system 100 and are not intended to limit the structure of the signal acquisition system 100. For example, the signal acquisition system 100 may include the first electrode set 12, the inertial sensor 150 disposed on the side of the first electrode set 12 away from the skin of the user, and the processing circuit 140 (e.g., without the second electrode set 13). As another example, as shown in FIG. 4 or FIG. 5, the signal acquisition system 100 may include the first electrode set 12, the second electrode set 13, the inertia sensor 150 disposed on the side of the first electrode away from the skin of the user, and the processing circuit 140. Descriptions regarding the inertial sensor 150 may be found in the related description of FIG. 1. As yet another example, the signal acquisition system 100 may include the first electrode set 12, the second electrode set 13, and the processing circuit 140 (e.g., without the inertial sensor 150).

FIG. 6 is a block diagram illustrating an exemplary signal acquisition system according to some embodiments of the present disclosure. As shown in FIG. 6, the signal acquisition system 300 may include a wearable body 310, a first electrode set 32, a second electrode set 33, and a processing circuit 340. The first electrode set 12 may include two first electrodes 320. The second electrode set 13 may include two second electrodes 330. The functions and structure distribution of the wearable body 310 and the first electrodes 320 illustrated in FIG. 6 may be similar to those described in FIGs. 1-5 of the wearable body 110 and the first electrode 120, respectively, and will not be repeated herein. The difference between the signal acquisition system 300 of FIG.6 and the signal acquisition system 100 described in FIGs. 1-5 is the functions and structure distribution of the second electrode 330. For example, the second electrode 330 is configured to acquire a detection signal reflecting a contact impedance between the two first electrodes 320 and the skin of the user, and accordingly, the processing circuit 340 is configured to eliminate motion artifacts from a physiological signal based on the detection signal (e.g., the detection signal and the physiological signal may be plugged into circuits to be processed in analog circuits, digital circuits, or algorithm). In some embodiments, the second electrodes 330 are configured to acquire the detection signal reflecting the contact impedance between the two second electrodes 330 and the skin of the user. To ensure the motion consistency between the first electrode 320 and the second electrode 330, in some embodiments, a minimum distance between an edge of each second electrode 330 and an edge of the first electrode 320 corresponding to the second electrode 330 is set to be less than 10 cm. In some embodiments, each second electrode 330 is inelastically connected to one first electrode 320, and a ratio of a difference to a movable distance of the first electrode 320 corresponding to the second electrode 330 on a surface parallel to the skin surface is not greater than 50%. The difference is a difference between the movable distance of the first electrode 320 and the movable distance of the second electrode 330 corresponding to the first electrode 320 on the surface parallel to the skin surface. As another example, to make the contact impedance between the two first electrodes 320 and the two second electrodes 330 and the skin as consistent as possible, the contact impedance of the second electrodes 330 may indirectly reflect the contact impedance of the first electrode 320, and each second electrode 330 may be identical to the first electrode 320 corresponding to the second electrode 330. For example, the conductivity of the material of each second electrode 330 and the material of the first electrode 320 corresponding to the second electrode 330 may be the same. As another example, a height of the protrusion of each second electrode 330 relative to the wearable body 310 may be the same as a height of the protrusion of the first electrode 320 corresponding to the second electrode 330 along a direction perpendicular to the skin of the user. As yet another example, the hardness and/or the folding degree of the material of each second electrode 330 may be the same as the hardness and/or the folding degree of the first electrode 320 corresponding to the second electrode 330. As yet another example, the contact area of each second electrode 330 with the skin may be the same as the contact area of the first electrode 320 corresponding to the second electrode 330 with the skin. In this way, the motion consistency and the contact impedance between the first electrode 320 and the second electrode 330 with the skin is as consistent as possible, and the detection signal of the contact impedance between the two second electrode 330 and the skin of the user acquired by the two second electrodes 330 may be used to reflect the detection signal of the contact impedance between the two first electrodes 320 and the skin of the user. In some embodiments, the processing circuit 340 may differentially process the signal acquired by the two first electrodes 320 to obtain the physiological signal, and the processing circuit 340 may differentially process the signal acquired by the two second electrodes 330 to obtain the detection signal and eliminate motion artifacts in the physiological signal based on the detection signal.

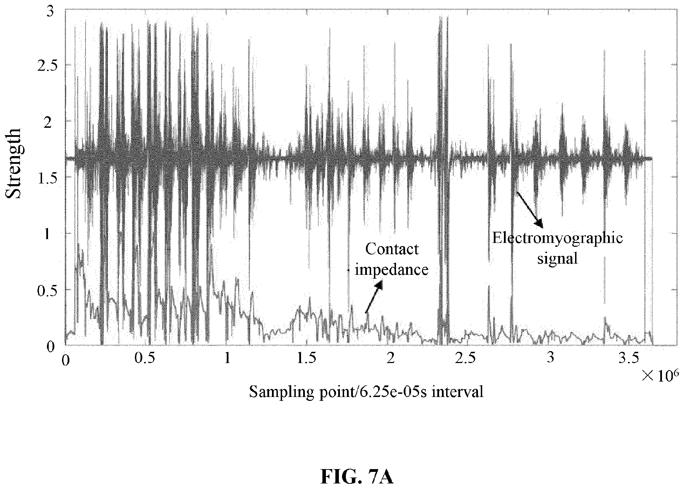

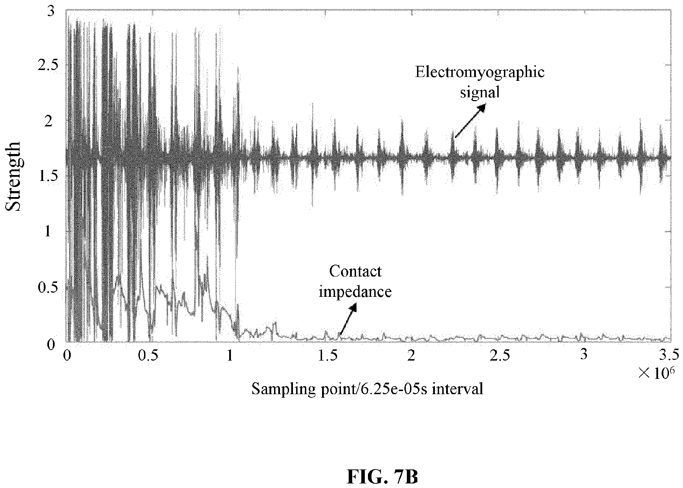

In some embodiments, the signal acquisition system 300 further includes an excitation source 370 electrically connected to the two second electrodes 330. The excitation source 370 may be configured to provide an excitation signal to generate a detection signal reflecting a contact impedance between the first electrode set 32 and the human body. In some embodiments, the excitation source 370 may be an alternating current excitation source, a direct current excitation source, or a combination thereof. Merely by way of example, the excitation signal may be understood to form a closed loop after flowing through the human body via the second electrode 330, at this point, the detection signal may correspond to a partial voltage of a contact impedance between the second electrode set 33 and the human body in that closed loop. FIG. 7A is a schematic diagram illustrating a fluctuation relationship between a contact impedance and an electromyographic signal according to some embodiments of the present disclosure. FIG. 7B is a schematic diagram illustrating a fluctuation relationship between a contact impedance and an electromyographic signal according to some embodiments of the present disclosure. As shown in FIGs. 7A and 7B, a value of contact impedance fluctuates correlatively with motion at the same sampling points regardless of whether the value of the contact impedance is large or small (e.g., the value of contact impedance increases with an increase in the EMG signal and decreases with a decrease in the EMG signal). Accordingly, motion artifacts may be obtained based on the contact impedance, and motion artifacts obtained (e.g., motion artifacts characterized by the detection signal reflecting the contact impedance between the first electrode set 32 and the skin of the user acquired by the second electrode set 33) may be utilized to eliminate motion artifacts in the physiological signal acquired by the first electrode set 32. For example, frequency analysis may be performed on the contact impedance to obtain a dominant frequency of the motion artifacts, and amplitude analysis (e.g., fluctuating value/neighborhood average, etc.) may be performed on the contact impedance to obtain information about the strength of the motion artifacts.

The excitation source 370 may be a circuit element that provides electrical energy. In some embodiments, the excitation source 370 may provide an excitation signal at a first frequency to generate a detection signal reflecting the contact impedance between the second electrode 330 and the human body. In some embodiments, the excitation source 370 may be a current source or a voltage source. It should also be noted that the setting of the intensity of the excitation source 370 also needs to consider a safe voltage or a safe current of the human body to ensure the safety of the human body, and the strength of the excitation source 370 should not be too high. In some embodiments, the current strength of the excitation source 370 may be less than 1 mA. Further, in some embodiments, the current strength of the excitation source 370 may be less than 100 µA. Further, in some embodiments, the current strength of the excitation source 370 may be 10 µA.

In some embodiments, a count of the excitation source 370 may be one, which may be connected to a plurality of second electrodes 330 via a plurality of circuit branches to provide the excitation signal to the plurality of second electrodes 330. Each of the plurality of circuit branches may be provided with a second electrode 330. In some embodiments, there may be a plurality of excitation sources, and each of the plurality of excitation sources 370 may be separately connected to one or more second electrodes 330 to provide the excitation signal to each second electrode 330.

In some embodiments, the excitation source 370 may simultaneously generate a plurality of excitation signals having different frequencies and may provide the excitation signals to a plurality of second electrodes 330 having the same frequency or different frequencies, thereby generating detection signals of different frequencies. For example, the excitation source 370 may provide the plurality of second electrodes 330 with excitation signals having different frequencies to acquire different detection signals. The plurality of second electrodes 330 may have a large distance (e.g., greater than or equal to 5 cm) from each other so that the closed loop through which the excitation signals flow may pass through different human tissues. Thus, the detection signals corresponding to the plurality of second electrodes 330 with different frequencies may be configured to reflect composition information of the human body, such as a body fat rate, a bone density, a body fluid content, etc. Specifically, the excitation source 370 may provide a second excitation signal at a second frequency that is different from the first frequency to generate a second detection signal. The second detection signal may reflect impedance information on a closed loop formed between the second electrode 330 and the human body at the second frequency. The impedance information includes contact impedance between the second electrode 330 and the human body, and impedance of the body tissue on the closed loop. In some embodiments, the composition information of the human body may be determined using a first detection signal generated by a first excitation signal (e.g., an excitation signal having the first frequency), and a second detection signal generated by a second excitation signal.

In some embodiments, the excitation signal may be a voltage signal or a current signal. In some embodiments, the excitation signal may be an alternating current signal having a first frequency such that the generated detection signal also has a first frequency. The first frequency may be set in a frequency range that is less susceptible to external interference (e.g., work frequency interference) and that interferes less with the physiological signal.

In some embodiments, the frequency of the excitation signal may be determined based on a frequency range of the physiological signal. In some embodiments, the frequency of the excitation signal may be higher than the frequency range of the physiological signal to avoid a large portion of the frequency band in which the physiological signal is located and reduce mutual interference between the physiological signal and the excitation signal. In some embodiments, the frequency of the excitation signal may be no less than 850 Hz. For example, the frequency of the excitation signal may be no less than 400 Hz. If the strength of the excitation signal is large or the contact impedance corresponding to the electrode at a site to be measured may be allocated with a sufficiently voltage, the physiological signal interfere with the excitation signal to a lesser extent, and the excitation signal (e.g., an excitation signal with a frequency that may be no less than 400 Hz) may tolerate a small portion of physiological signal (e.g., a physiological signal with a frequency within a range of 400 Hz to 850 Hz). In some embodiments, since the frequency of the physiological signal (e.g., the EMG signal) is mainly concentrated in a range of 20 Hz to 400 Hz, and the lower frequency band has motion artifacts of greater intensity, the frequency of the excitation signal provided by the excitation source 370 may be greater than 250 Hz to avoid being affected by the EMG signal and the motion artifacts. In some embodiments, the frequency of the excitation source may also avoid a frequency range in which a work frequency noise is located, since there is also interference from the work frequency (e.g., 50 Hz or 60 Hz alternating current power supply and harmonics thereof) noise in the signal acquisition system 300. In some embodiments, a difference between the frequency of the excitation signal provided by the excitation source 370 and an integer multiple of 50 Hz or 60 Hz may be no less than 1 Hz, or the difference between the frequency of the excitation signal provided by the excitation source 370 and an integer multiple of 50 Hz or 60 Hz may be no less than 2%. In some embodiments, the frequency of the excitation signal provided by the excitation source 370 may be 400 Hz or more, such as 460 Hz, 640 Hz, 830 Hz, or the like. In some embodiments, the setting of the frequency of the excitation signal provided by the excitation source 370 also needs to be considered in conjunction with the sampling frequency of the processing circuit 340. Specifically, in order to acquire the detection signal, the sampling frequency may be at least two times and more than the frequency of the excitation signal provided by the excitation source 370. To minimize errors, in some embodiments, the sampling frequency may be 4 times and more than the frequency of the excitation signal provided by the excitation source 370. It should be noted that since a count of channels to be sampled may be large and most processing circuits 340 have difficulty in supporting extremely high sampling frequencies for a plurality of channels, the sampling frequency of the processing circuits 340 and the frequency of the excitation signal provided by the excitation source 370 should not be too high. Based on this, in some embodiments, the frequency of the excitation signal provided by the excitation source 370 may be set within a range of 250 Hz to 2000 Hz.

In some embodiments, the signal acquisition system 300 may acquire the physiological signal and the detection signal separately during different time periods. Exemplarily, the signal acquisition system 300 may acquire the physiological signal during a first time period. Exemplary, the signal acquisition system 300 may acquire the detection signal during a second time period. For example, the signal acquisition system 300 may include a switching circuit, and the switching circuit may be configured to control a conductive state of the first electrode set with the processing circuit 340, and may also be configured to control the conductive state of the second electrode set with the excitation source 370 so that only the first electrode set remains in a conductive state with the processing circuit 340 or only the second electrode set remains in a conductive state with the excitation source 370 at the same moment. In this way, by time-sharing acquisition and processing of the detection signal and the physiological signal, the processing pressure of the device may be reduced, and computational resources may be saved while avoiding the signals from interfering with each other. In some embodiments, the signal acquisition system 300 may acquire the physiological signal and the detection signal at the same time and transmit them to different processing circuits through different transmission channels to avoid signals from interfering with each other.

FIG. 8 is a block diagram illustrating an exemplary signal acquisition system 400 according to some embodiments of the present disclosure. As shown in FIG. 8, the signal acquisition system 400 may include a wearable body 410, two electrodes 420, a processing circuit 440, and an alternating current excitation source 470. The wearable body 410, the processing circuit 440, and the alternating current excitation source 470 illustrated in FIG. 8 are similar to the wearable body 310, and the processing circuit 340 illustrated in FIG. 6, and will not be repeated herein. The signal acquisition system 400 of FIG. 8 differs from the signal acquisition system 300 of FIG. 6 in that the signal acquisition system 400 includes only one set of electrodes (two electrodes 420), and the two electrodes 420 may acquire both the physiological signal and the detection signal reflecting the contact impedance between the two electrodes 420 and the skin. The excitation source in the signal acquisition system 400 is the alternating current excitation source 470, while the excitation source in the system 300 may be an alternating current excitation source, a direct current excitation source, or a combination thereof. In some embodiments, the two electrodes 420 for acquiring the physiological signal and the detection signal may be fixed on the wearable body 410 and in contact with the skin of the user.

In some embodiments, the alternating current excitation source 470 is electrically connected to the two electrodes 420. The alternating current excitation source 470 is configured to provide an excitation signal to generate a detection signal reflecting the contact impedance between the two electrodes 420 and the human body. Merely by way of example, the excitation signal may be understood as forming a closed loop after flowing through the human body via the electrodes 420. At this point, the detection signal may correspond to the partial voltage of the contact impedance between the electrodes 420 and the human body in this closed loop. In some embodiments, as shown in FIGs. 7A and 7B, the value of the contact impedance fluctuates in correlation with movement (e.g., the value of the contact impedance increases with an increase in the EMG signal and decreases with a decrease in the EMG signal). Thus, motion artifacts may be obtained based on the contact impedance. For example, the motion artifacts may be eliminated from the physiological signal acquired by the two electrodes 420 using the motion artifacts (e.g., motion artifacts are characterized by the detection signal reflecting the contact impedance between the two electrodes 420 and the skin of the user acquired by the two electrodes 420). It should be understood that the frequency of the physiological signal, the frequency of the excitation signal, or the like, acquired by the signal acquisition system 400 shown in FIG. 8 may be similar to those of the signal acquisition system 300 shown in FIG. 6, and are not described herein. Since the physiological signal and the detection signal are acquired by the same set of electrodes, the signal acquisition system 400 may acquire the physiological signal and the detection signal separately at different time periods. Exemplarily, the signal acquisition system 400 may acquire the physiological signal during a first time period. The signal acquisition system 400 may acquire the detection signal during a second time period. For example, the signal acquisition system 400 may include switching circuit, the switching circuit may be configured to control the conductive state of the two electrodes 420 with the processing circuit 440, and may also be configured to control the conductive state of the two electrodes 420 with the excitation source 370 so that the two electrodes 420 in the conductive state with the processing circuit 440 or the two electrodes 420 in the conductive state with the alternating current excitation source 470 at the same moment. In this way, by time-sharing acquisition and processing of the detection signal and the physiological signal, the processing pressure of the device may be reduced, and computational resources may be conserved while avoiding the signals from interfering with each other.

It should be noted that the above description of the signal acquisition system is only exemplary and does not limit the present disclosure to the scope of the cited embodiments. The beneficial effects that may be generated by different embodiments may vary. In different embodiments, the beneficial effects that may be generated may be any one or a combination of the above, or any other possible beneficial effects.

The present disclosure has the following technical effects. First, through the differentiated design of the first electrode set and the second electrode set, differentiation of the physiological signals acquired by the first electrode set and the second electrode set may be realized. Second, through the differentiated design of the two second electrodes in the second electrode set, differentiated physiological signals may be realized. Third, the differentiated physiological signals may reduce the interference of motion artifacts, so as to extract the physiological signals with higher quality. Fourth, due to the correlation between the contact impedance and the motion, the interference of motion artifacts in the physiological signal are eliminated based on the contact impedance.

Having thus described the basic concepts, it may be rather apparent to those skilled in the art after reading this detailed disclosure that the foregoing detailed disclosure is intended to be presented by way of example only and is not limiting. Although not explicitly stated here, those skilled in the art may make various modifications, improvements, and amendments to the present disclosure. These alterations, improvements, and amendments are intended to be suggested by this disclosure and are within the spirit and scope of the exemplary embodiments of the present disclosure.

Moreover, certain terminology has been used to describe embodiments of the present disclosure. For example, the terms "one embodiment", "an embodiment", and/or "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of the present disclosure are not necessarily all referring to the same embodiment. In addition, some features, structures, or characteristics of one or more embodiments in the present disclosure may be properly combined.

Furthermore, the recited order of processing elements or sequences, or the use of numbers, letters, or other designations, therefore, is not intended to limit the claimed processes and methods to any order except as may be specified in the claims. Although the above disclosure discusses some embodiments of the invention currently considered useful by various examples, it should be understood that such details are for illustrative purposes only, and the additional claims are not limited to the disclosed embodiments. Instead, the claims are intended to cover all combinations of corrections and equivalents consistent with the substance and scope of the embodiments of the present disclosure. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that object of the present disclosure requires more features than the features mentioned in the claims. Rather, claimed subject matter may lie in less than all features of a single foregoing disclosed embodiment.

In some embodiments, the numbers expressing quantities or properties used to describe and claim certain embodiments of the present disclosure are to be understood as being modified in some instances by the term "about", "approximate", or "substantially". For example, "about", "approximate", or "substantially" may indicate ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the present disclosure are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes. History application documents that are inconsistent or conflictive with the contents of the present disclosure are excluded, as well as documents (currently or subsequently appended to the present specification) limiting the broadest scope of the claims of the present disclosure. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the present disclosure disclosed herein are illustrative of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A signal acquisition system, comprising:
a wearable body;
a plurality of electrodes fixed on the wearable body and in contact with the skin of a user, wherein the plurality of electrodes include a first electrode set and a second electrode set, the first electrode set is configured to acquire a physiological signal and the second electrode set is configured to acquire a detection signal; and
a processing circuit configured to eliminate, based on the detection signal, motion artifacts in the physiological signal.

2. The signal acquisition system of claim 1, wherein the first electrode set includes two first electrodes arranged at interval, and the second electrode set includes two second electrodes arranged close to the two first electrodes, respectively, the physiological signal is a first physiological signal, the detection signal is a second physiological signal, and the processing circuit is configured to eliminate, based on the second physiological signal, the motion artifacts in the first physiological signal.

3. The signal acquisition system of claim 2, wherein conductivities of materials of the two second electrodes are different.

4. The signal acquisition system of claim 2 or 3, wherein heights of protrusions of the two second electrodes relative to the skin of the user are different along a direction perpendicular to the skin surface of the user.

5. The signal acquisition system of any one of claims 2-4, wherein hardness of the materials of the two second electrodes are different, or
folding degrees of the materials of the two second electrodes are different.

6. The signal acquisition system of any one of claims 2-5, wherein areas of the two second electrodes are different.

7. The signal acquisition system of claim 1, wherein the first electrode set includes two first electrodes arranged at interval, and the second electrode set includes a second electrode arranged close to one of the two first electrodes, the second electrode and the one of the two first electrodes close to the second electrode are configured to acquire the detection signal.

8. The signal acquisition system of any one of claims 2-7, wherein a conductivity of a material of each second electrode is weaker than a conductivity of a material of a first electrode corresponding to the second electrode.

9. The signal acquisition system of any one of claims 2-8, wherein along a direction perpendicular to the skin surface of the user, a height of a protrusion of each second electrode relative to the wearable body is less than a height of a protrusion of a first electrode corresponding to the second electrode relative to the wearable.

10. The signal acquisition system of any one of claims 2-9, wherein a hardness of a second material of each second electrode is greater than a first material of a first electrode corresponding to the second electrode, or
a folding degree of the second material of each second electrode is greater than a folding degree of the first material of the first electrode corresponding to the second electrode.

11. The signal acquisition system of any one of claims 2-10, wherein a contact area of each second electrode with the skin is less than a contact area of a first electrode corresponding to the second electrode with the skin.

12. The signal acquisition system of claim 1, wherein the first electrode set includes two first electrodes arranged at interval, and the second electrode set includes two second electrodes arranged close to the two first electrodes, respectively, and the detection signal reflects a contact impedance between the two first electrodes and the skin of the user.

13. The signal acquisition system of claim 12, further comprising an excitation source electrically connected to the two second electrodes, wherein the excitation source is configured to provide an excitation signal.

14. The signal acquisition system of claim 13, wherein a frequency of the physiological signal is within a range of 20 Hz-400 Hz, and a frequency of the excitation signal is not less than 250 Hz.

15. The signal acquisition system of claim 14, wherein a difference between the frequency of the excitation signal and an integer multiple of 50 Hz is not less than 1 Hz; or
a difference between the frequency of the excitation signal and an integer multiple of 60 Hz is not less than 1 Hz.

16. The signal acquisition system of any one of claims 12-15, wherein the frequency of the excitation signal is higher than a frequency range of the physiological signal.

17. The signal acquisition system of claim 12, wherein the detection signal and the physiological signal are acquired separately at different time periods.

18. The signal acquisition system of any one of claims 2-17, wherein a minimum distance between an edge of each second electrode and an edge of a first electrode corresponding to the second electrode is less than 10 cm.

19. The signal acquisition system of any one of claims 2-18, wherein each second electrodes is inelastically connected to a first electrode corresponding to the second electrode, and
a ratio of a difference to a movable distance of the first electrode corresponding to the second electrode on a surface parallel to the skin surface is not greater than 50%, the difference being a difference between a movable distance of the second electrode and the movable distance of the first electrode corresponding to the second electrode on the surface parallel to the skin surface.

20. The signal acquisition system of any one of claims 1-19, wherein the processing circuit is configured to:
obtaining the physiological signal by differentially processing signals acquired by the two first electrodes;
obtaining the detection signal by differentially processing signals acquired by the two second electrodes; and
eliminating, based on the detection signal, the motion artifacts in the physiological signal.

21. The signal acquisition system of any one of claims 1-20, further comprising an inertial sensor, wherein the inertial sensor is arranged on a side of the first electrode set depart from the skin of the user, and the inertial sensor is configured to measure motion artifacts of the first electrode set.

22. A signal acquisition system, comprising:
a wearable body;
a plurality of electrodes fixed on the wearable body and in contact with the skin of a user, wherein the plurality of electrodes include two electrodes arranged at interval for acquiring a physiological signal;
an excitation source electrically connected to the two electrodes, wherein the excitation source is configured to provide an excitation signal for generating a detection signal reflecting a contact impedance between the two electrodes and the skin of the user; and
a processing circuit configured to eliminate, based on the detection signal, motion artifacts in the physiological signal.

23. The signal acquisition system of claim 22, wherein a frequency of the physiological signal is within a range of 20 Hz-400 Hz, and a frequency of the excitation signal is not less than 250 Hz.

24. The signal acquisition system of claim 22, wherein a difference between a frequency of the excitation signal and an integer multiple of 50 Hz is not less than 1 Hz; or
a difference between the frequency of the excitation signal and an integer multiple of 60 Hz is not less than 1 Hz.

25. The signal acquisition system of any one of claims 22-24, wherein a frequency of the excitation signal is higher than a frequency range of the physiological signal.

26. The signal acquisition system of claim 22, wherein the detection signal and the physiological signal are acquired separately at different time periods.
